# EUROPEAN PATENT SPECIFICATION

(11) **EP 2 455 063 B1**
(45) Date of publication and mention of the grant of the patent: **14.12.2016**
(21) Application number: 10191786.2
(22) Date of filing: 18.11.2010
(51) Int. Cl.: A61K 8/44, A61K 8/46, A61K 8/81, A61K 8/92, A61Q 5/12, A61Q 19/10

(54) **Personal cleansing composition comprising highly nourishing oils**
Körperreinigungszusammensetzung mit stark pflegenden Ölen
Composition de nettoyage personnel comprenant des huiles très nourrissantes

(43) Date of publication of application: 23.05.2012
(73) Proprietor: Dalli-Werke GmbH & Co. KG, 52224 Stolberg (DE)
(72) Inventor: Bastian, Christin, 55246 Mainz-Kostheim (DE); Betsch, Roland, 67269 Grünstadt (DE); Begoin-Petreins, Britta, 33034 Brakel (DE); Böll, Liz, 67278 Bockenheim (DE); Lähn, Natascha, 67591 Offstein (DE); Schimmele, Stefan, 68782 Brühl (DE); Nadeem, Zia Ullah, 55232 Alzey (DE)
(74) Representative: f & e patent

(56) References cited:
- WO-A1-97/32568
- WO-A1-03/074021
- DE-A1-102008 046 178
- FR-A1- 2 892 302
- FR-A1- 2 912 914
- US-A1- 2007 286 838
- Lubrizol: "Almond rich moisturizing shower creme CL-B0045(EU)", , 26 April 2010 (2010-04-26), XP002635109, Retrieved from the Internet: URL:http://www.lubrizol.com/PersonalCare/C L-B0045(EU)-Almond-Rich-Moisturizing-Showe r-Creme.pdf [retrieved on 2011-05-03]
- STUSSI I ET AL: "ARGANIA SPINOSA - HOW ECOLOGICAL FARMING, -AIR TRADE AND SUSTAINABILITY CAN DRIVE THE RESEARCH FOR NEW COSMETIC ACTIVE INGREDIENTS", SOFW-JOURNAL SEIFEN, OELE, FETTE, WACHSE, VERLAG FUR CHEMISCHE INDUSTRIE, AUGSBURG, DE, vol. 131, no. 10, 1 January 2005 (2005-01-01), pages 46,48,50-52,54, XP009077275, ISSN: 0942-7694
- Lubrizol: "Carbopol Ultrez 20 Polymer", Lubrizol Technical Data Sheet 332, 5 June 2006 (2006-06-05), XP002635110, Retrieved from the Internet: URL:http://www.lubrizol.com/WorkArea//Down loadAsset.aspx?id=32000 [retrieved on 2011-05-03]

## Description

The present invention relates to personal cleansing compositions, preferably shower gels, comprising marula oil .

Marula oil is a rare and expensive vegetable oil that has been known for its beneficial effect on the human skin and hair. This oil has been used in traditional natural medicine and cosmetics as well as in modern cosmetics, predominantly in care products such as creams and lotions to be applied to the skin. This oil has highly nourishing properties improving the structure of the skin and/or imparting a comfortable feeling on the skin. Thus, there is a desire to incorporate this oil into personal cleansing compositions allowing the user to benefit from its unique skin and hair care properties by just applying a cleansing product to the skin or hair.

However, cleansing compositions are generally aqueous systems and it has been proven very difficult to provide stable aqueous compositions comprising the above nourishing oil.

DE 10 2008 037 633 A1 is directed to cosmetic compositions comprising at least 0.01 % by weight of one or more oils derived from a sumac plant such as marula oil and at least 0.01 % by weight of an ester oil. There are described various kinds of cosmetic compositions and the majority of the numerous examples relates to products for conditioning the skin or hair, especially for restructuring the hair. Nevertheless, one exemplary composition is a shower gel comprising 2.5 % by weight of marula oil and 0.5 % of Cetiol® HE (polyoxyethylene glyceryl monococoate; INCI name: PEG-7 Glycerol Cocoate). Ester oils are synthetic lipids which are prepared by first cleaving the triglycerides of vegetable oils into fatty acids and glycerol and then esterifying the fatty acids selectively with glycerol, saccharose, glucose or other alcohols to obtain a new and intended combination of fatty acid and alcohol. The term "ester oils" also includes PEG-7 Glycerol Cocoate and similar esters that are alkoxylated at the alcohol or ester part or both. Ester oils in general, much more alkoxylated ester oils are not undisputed as regards their effect on the human skin. Moreover, their production is costly as the transesterification occurs at about 160 to 200°C.

Thus, it is the object of the present invention to provide a personal cleansing composition comprising a highly nourishing oil and being stable without the necessity to incorporate synthetic ester oils.

The object it met by a personal cleansing composition comprising:
(a) 0.01 to 15 % by weight of a nourishing oil component which is a vegetable oil having an oleic acid content of at least 38 % by weight (based on the total amount of esterified fatty acids in the vegetable oil) and which is marula oil, wherein when one or more of apricot kernel oil, argan oil and tamanu oil are present the total amount of marula oil, apricot kernel oil, argan oil, and tamanu oil is 0.01 to 15 % by weight;
(b) 0.01 to 20 % by weight of a compatibilizing oil component which is a vegetable oil or mixture of vegetable oils being selected from soybean oil, olive oil, coconut oil, and sunflower oil,
   wherein the total amount of nourishing oil component and compatibilizing oil component does not exceed 25 % by weight;
(c) 5 to 50 % by weight of a surfactant system comprising:
   (i) an anionic surfactant or mixture of anionic surfactants; and
   (ii) an amphoteric and/or zwitterionic surfactant or mixtures thereof;
(d) 0.01 to 5.0 % by weight of hydrophobically modified crosslinked acrylic copolymer; and
(e) water,
   wherein all weight percentages of the components are based on the total weight of the cleansing composition and water is present in an amount adding up to 100 % by weight.

Marula oil (Sclerocarya Birrea Oil) is extracted, preferably by simple pressing filtration techniques without the use of solvents, from the seeds of the marula fruit tree native to Southern and Central Africa. Apricot kernel oil (Prunus Armeniaca Kernel Oil) is obtained from the kernels of the apricot by mechanical pressing. Argan oil (Argania Spinosa Oil) is obtained form the seeds of the argan tree (Argania spinosa L. Skeel) which is endemic to the arid southwestern regions of Morocco. Traditionally it is extracted mechanically. Tamanu oil (Calophyllum Inophyllum Seed Oil) is extracted from the kernel of the nuts of the tamanu tree through a pressing process. The tamanu tree is native to both the Pacific and Asian tropical regions.

All four nourishing oils have a high content of oleic acid, i.e. of at least 38 % by weight. Representative fatty acid distributions of the oils are as follows:

| | **Marula oil** | **Apricot kernel oil** | **Argan oil** | **Tamanu oil** |
|---|---|---|---|---|
| Palmitic acid (C16:0) | 11 % | 5 - 6 % | 10 - 15 % | 10 - 20 % |
| Palmitoleic acid (C16:1) | - | 0.2 - 1 % | - | |
| Stearic acid (C18:0) | 7 % | 1 - 2 % | 3 - 8 % | 10 - 20 % |
| Oleic acid (C18:1) | 75 % | 60 - 70 % | 38 - 50 % | 40 - 50 % |
| Linoleic acid (C18:2) | 4 - 6 % | 27 - 29 % | 30 - 40 % | 20 - 30 % |
| Linolenic acid (C18:3) | 0 - % | 0 - % | 0 - 5 % | 0 - 0.5 % |
| Arachic acid (C20:0) | | | 1 % | |
| Gadoleic acid (C20:1) | | | 1 % | |

All values are given in % by weight based on the total amount of fatty acids of the triglycerides present in the corresponding oil. They are only approximate values and may vary due to the fact that the oils are of natural origin.

In addition to the triglycerides the above nourishing oils comprise unsaponificables including phytochemicals such as vitamins (e.g. vitamin E), sterols, fatty alcohols, hydrocarbons such as sqaulenes and carotenoids. The phytochemicals add to the beneficial effects of the triglycerides. Although the amount of unsaponificables varies and depends on the type of vegetable oil it is generally within the range of from 0.5 to 2 % by weight based on the weight of the vegetable oil.

Marula oil, apricot kernel oil, argan oil, and tamanu oil may be used herein as refined or unrefined oils. Marula oil and apricot kernel oil are typically used as refined oils. Argan oil and tamanu oil are typically used as unrefined oils.

The nourishing oils are used in the present cleansing compositions as the nourishing oil component which consists of marula oil or its mixture with one, two, or all three nourishing oils. The nourishing oil component is present in the cleansing composition in an amount of from 0.01 to 15 % by weight, preferably from 0.02 to 10 % by weight, more preferably from 0.03 to 5 % by weight, and most preferably from 0.05 to 3 % by weight, each based on the total weight of the cleansing composition. This means that when more than one of marula oil, apricot kernel oil, argan oil and tamanu oil are present their total amount is within the above identified ranges.

The inventors of the present invention found that these highly nourishing oils can be formulated to stable aqueous cleansing compositions if used together with certain compatibilizing oils and a hydrophobically modified acrylic polymer.

The compatibilizing oil component of the present invention comprises one or more of soybean oil (Glycine Soja (Soybean) Oil), coconut oil (Cocos Nucifera (Coconut) Oil), sunflower oil (Helianthus Annuus (Sunflower) Seed Oil), and olive oil (Olea Europaea (Olive) Fruit Oil). Soybean oil has an oleic acid content of from 17 to 30 % by weight, coconut oil of from 5 to 10 % by weight, sunflower oil of from 14 to 39.4 % by weight, and olive oil of from 55 to 83 % by weight, each based on the total amount of fatty acids of the triglycerides present in the corresponding oil. Soybean oil, coconut oil, and sunflower oil have an oleic acid content of less than 39.5 % by weight. All these compatibilizing oils are common and inexpensive vegetable oils that are readily available throughout the world. Their extractions are well-known and need not be explained in detail. Like the above described nourishing oils the compatibilizing vegetable oil also contain phytochemicals having beneficial properties. As the compatibilizing vegetable oils also exhibit an advantageous conditioning effect on the skin, they add to the benefits of the nourishing oils.

Soybean oil, olive oil, coconut oil, and sunflower oil may be used herein as refined or unrefined oils. Soybean oil, olive oil, coconut oil, and sunflower oil are typically used as refined oils.

The compatibilizing oils are used in the present cleansing compositions as the compatibilizing oil component which consists of a single compatibilizing oil or a mixture of two, three or all four compatibilizing oils. The compatibilizing oil component is present in the coating composition in an amount of from 0.01 to 20.0 % by weight, preferably from 0.1 to 15.0 % by weight, more preferably from 0.5 to 10 % by weight, even more preferably from 1 to 8 % by weight, and most preferably from 2 to 5.5 % by weight, each based on the total weight of the cleansing composition. This means that when more than one of soybean oil, olive oil, coconut oil, and sunflower oil are present their total amount is within the above identified ranges.

The weight ratio of nourishing oil component to compatibilizing oil component typically ranges from 1:700 to 10:1 preferably ranges from 1:500 to 4:1, more preferably from 1:200 to 3:1, even more preferably from 1:150 to 2:1, still more preferably from 1:120 to 3:2, and most preferably from 1:100 to 1:1. In certain embodiments the weight ratio of nourishing oil component to compatibilizing oil component ranges from 1:100 to 1:2 or from 1:100 to 1:4 or from 1:100 to 1:10 or from 1:100 to 1:45.

The total amount of the nourishing oil component and the compatibilizing oil component does not exceed 25 % by weight, preferably it does not exceed 21 % by weight, more preferably it does not exceed 20 % by weight. The total amount typically ranges from 0.02 to 25 % by weight, preferably from 0.1 to 21 % by weight, more preferably from 0.1 to 20 % by weight, still more preferably from 0.5 to 15 % by weight, even more preferably from 1 to 10 % by weight, and most preferably from 2 to 8 % by weight, each based on the total weight of the cleansing composition. In embodiments where further vegetable oils in addition to the vegetable oils described herein are present the total amount of all vegetable oils is preferably within the limits defined above.

The hydrophobically modified crosslinked acrylic copolymer for use in the present cleansing composition is any crosslinked acrylic polymer which carries hydrophobic residues, preferably higher alkyl residues such C₅ to C₅₀ alkyl residues, more specifically C₁₀ to C₃₀ alkyl residues, along the polymer chain.

Those alkyl residues are typically bond to the polymer chain via ester bonds. The term "hydrophobically modified crosslinked acrylic copolymer" also includes mixtures or blends of polymers. In preferred embodiments the hydrophobically modified crosslinked acrylic copolymer is a crosslinked copolymer of (i) acrylic acid and/or methacrylic acid, and (ii) alkyl acrylates and/or alkyl methacrylates. In some embodiments the hydrophobically modified crosslinked acrylic copolymer is a crosslinked copolymer of acrylic acid, methacrylic acid, acrylates and methacrylates wherein the acrylate and methacrylate units of the polymer comprise the hydrophobic residues as defined above, i.e. the (meth)acrylates are typically C₅ to C₅₀ alkyl (meth)acrylates, preferably C₁₀ to C₃₀ alkyl (meth)acrylates. More preferably, the hydrophobically modified crosslinked acrylic copolymer is a crosslinked copolymer of acrylic acid, acrylates and methacrylates wherein the acrylate and methacrylate units of the polymer comprise the hydrophobic residues as defined above, i.e. the (meth)acrylates are typically C₅ to C₅ₒ alkyl (meth)acrylates, preferably C₁₀ to C₃₀ alkyl (meth)acrylates.

The type of crosslinking within the hydrophobically modified crosslinked acrylic copolymer is not essential. Typically, the hydrophobically modified crosslinked acrylic copolymer, including the preferred polymers described above, is crosslinked with polyalkenyl ethers, e.g. allyl ethers of pentaerythritol and/or allylethers of saccharose.

Preferably, the personal cleansing compositions of the present invention comprise a hydrophobically modified crosslinked acrylic copolymer which is known under the INCI name Acrylates/C10-30 Alkyl Acrylates Crosspolymer. More preferably, the backbone of the hydrophobically modified crosslinked acrylic copolymer can be depicted by the following formula (crosslinks not shown): 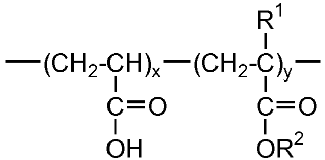 wherein R¹ is H or CH₃ and R² is C₁₀-C₃₀ alkyl, i.e. the copolymer comprises units derived from acrylic acid, C₁₀-C₃ₒ alkyl acrylate and C₁₀-C₃₀ alkyl methacrylate. It preferably is crosslinked with a polyalkenyl ether.

Typically, the hydrophobically modified crosslinked acrylic copolymer has a very high molecular weight which cannot be determined by conventional techniques. A single molecule may have a molecular weight of up to several billion Daltons.

Examples of commercially available hydrophobically modified crosslinked acrylic copolymer for use in the present cleansing compositions include Carbopol® 1342 Polymer, Carbopol® 1382 Polymer, Carbopol® ETD 2020 Polymer, Carbopol® Ultrez 20 Polymer, Carbopol® Ultrez 21 Polymer, Pemulen® TR-2 Polymer and Pemulen® TR-1 Polymer with Carbopol® 1342 Polymer, Carbopol® 1382 Polymer, Carbopol® ETD 2020 Polymer, and Carbopol® Ultrez 20 Polymer being preferred and Carbopol® ETD 2020 Polymer and Carbopol® Ultrez 20 Polymer being most preferred. All Carbopol® and Pemulen® Polymers are available from Lubrizol Advanced Materials, Inc.

If the hydrophobically modified crosslinked acrylic copolymer contains acid groups such as carboxyl groups in the preferred polymers mentioned above the addition of an alkaline material may become necessary to adapt the pH value of the cleansing composition. Examples of alkaline materials include sodium hydroxide, ammonium hydroxide, potassium hydroxide, L-arginine, aminomethyl propanol, tetrahydroxypropyl ethylenediamine, triethanolamine, Tromethamine (1,3-propanediol, 2-amino-2-(hydroxymethyl)-), PEG-15 Cocamine, diisopropanolamine, and triisopropanolamine.

The total amount of any hydrophobically modified crosslinked acrylic copolymer in the cleansing compositions ranges from 0.01 to 5 % by weight, preferably from 0.05 to 4 % by weight, more preferably from 0.1 to 3 % by weight, even more preferably from 0.5 to 2 % by weight, still more preferably from 0.6 to 1.5 % by weight, and most preferably from 0.7 to 1.3 % by weight, each based on the total weight of the cleansing composition.

The surfactant system of the present cleansing composition comprises:
(i) one or more anionic surfactants;
(ii) one or more amphoteric and/or zwitterionic surfactants; and
(iii) optional one or more nonionic surfactants.

The surfactant systems comprises 5 to 50 % by weight, preferably 5.5 to 40 % by weight, more preferably 6 to 30 % by weight, even more preferably 7 to 23 % by weight, still more preferably 8.5 to 17 % by weight, and most preferably 10 to 15 % by weight of the total cleansing composition.

### (i) Anionic surfactants

The anionic surfactant may be, for example, an alkyl sulfate (e.g. C₁₂-C₁₈ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 18 carbons, preferably 12 to 18 carbons, more preferably 12 carbons, n has an average value of greater than 1.0, preferably between 2 and 4; and M is a solubilizing cation such as sodium, potassium, magnesium, ammonium or substituted ammonium. Magnesium, ammonium and sodium lauryl ether sulfates are preferred. Sodium lauryl ether sulfate (Sodium Laureth Sulfate) is especially preferred.

The anionic surfactant may also be an aliphatic sulfonate, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or an aromatic sulfonate such as alkyl benzene sulfonate.

The anionic surfactant may also be acyl glutamates, sulfosuccinates such as alkyl sulfosuccinates (including mono- and dialkyl, e.g., C₆-C₂₂ sulfosuccinates); alkyl and acyl taurates, alkyl and acyl sarcosinates, sulfoacetates, C₈-C₂₂ alkyl phosphates and phosphates, alkyl phosphate esters and alkoxyl alkyl phosphate esters, acyl lactates, C₈-C₂₂ monoalkyl succinates and maleates, sulphoacetates, and acyl isethionates.

Acyl glutamates are known anionic surfactants corresponding to the formula: 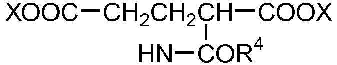 in which R⁴CO is a linear or branched acyl group containing 6 to 22 carbon atoms and 0, 1, 2 or 3 double bonds and X is hydrogen, an alkali metal and/or alkaline earth metal, ammonium, alkylammonium, alkanolammonium or glucammonium. They are produced, for example, by Schotten-Baumann acylation of glutamic acid with fatty acids, fatty acid esters or chlorides. An overview of the production and properties of acyl glutamates was published by M. Takehara et al. in J. Am. Oil. Chem. Soc., 49, (1972) 143. Typical examples of acyl glutamates suitable for the purposes of the invention are those derived from fatty acids containing 6 to 22 and preferably 12 to 18 carbon atoms. Coconut oil fatty acid glutamates, for example of C_{12/14} or C_{12/18} coconut oil fatty acid glutamate, are particularly preferred. The mono- or dialkali metal salts of acyl glutamate in particular are used.

Sulfosuccinates may be monoalkyl sulfosuccinates having the formula:

R⁴O₂CCH₂CH(SO₃M)CO₂M;

amido-MEA sulfosuccinates of the formula:

R⁴CONHCH₂CH₂O₂CCH₂CH(SO₃M)CO₂M,

wherein R⁴ ranges from C₈-C₂₂ alkyl and M is a solubilizing cation;
amido-MIPA sulfosuccinates of the formula:

RCONH(CH₂)CH(CH₃)(SO₃M)CO₂M

where M is as defined above.

Also included are the alkoxylated citrate sulfosuccinates; and alkoxylated sulfosuccinates such as the following: 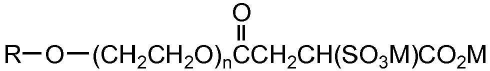 wherein n = 1 to 20; and M is as defined above.

Sarcosinates are generally indicated by the formula:

RCON(CH₃)CH₂CO₂M,

wherein R ranges from C₈ to C₂₀ alkyl and M is a solubilizing cation.

Taurates are generally identified by the formula:

R²CONR³CH₂CH₂SO₃M

wherein R² ranges from C₈-C₂₀ alkyl, R³ ranges from C₁-C₄ alkyl and M is a solubilizing cation.

Another class of anionics are carboxylates such as follows:

R-(CH₂CH₂O)ₙCO₂M or R-O-(CH₂CH₂O)ₙ-CH₂-CO₂M

wherein R is C₈ to C₂₀ alkyl; n is 0 to 20; and M is as defined above.

Another carboxylate which can be used is an amido alkyl polypeptide carboxylate such as, for example, Monteine LCQ by Kreglinger Europe NV.

Another anionic surfactant which may be used are the C₈-C₁₈ acyl isethionates. These esters are prepared by reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

Acyl isethionates, when present, will generally range from about 0.5 to 15% by weight of the total composition. When present, this component is preferably present from about 1 to about 10%. The acyl isethionate may be an alkoxylated isethionate such as is described in Ilardi et al., U.S. Patent No.5,393,466. This compound has the general formula: 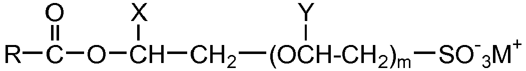 wherein R is an alkyl group having 8 to 18 carbons, m is an integer from 1 to 4, X and Y are hydrogen or an alkyl group having 1 to 4 carbons and M⁺ is a monovalent cation such as, for example, sodium, potassium or ammonium.

The preferred anionic surfactants for use in the present cleansing composition are alkyl sulfates, alkyl ether sulfates, sulfosuccinates, and/or acyl glutamates, the alkyl ether sulfates (e.g. Sodium Laureth Sulfate) being most preferred.

Typically, the anionic surfactant(s), including the preferred ones described above, comprise(s) from about 4.9 to 30 % by weight, preferably from 5 to 25 % by weight, more preferably from 5 to 20 % by weight, even more preferably from 6 to 15 % by weight, and most preferably from 7 to 12 % by weight of the total cleansing composition.

### (ii) Zwitterionic and amphoteric surfactants:

Zwitterionic surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. A general formula for these compounds is: 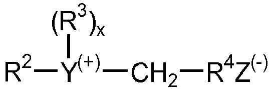 wherein R² contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms; R³ is an alkyl or monohydroxyalkyl group containing about 1 to about 3 carbon atoms; X is 1 when Y is a sulfur atom, and 2 when Y is a nitrogen or phosphorus atom; R⁴ is an alkylene or hydroxyalkylene of from about 1 to about 4 carbon atoms and Z is a radical selected from the group consisting of carboxylate, sulfonate, sulfate, phosphonate, and phosphate groups.

Examples of such surfactants include:
4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate;
3-[P,P-diethyl-P-3,6,9-trioxatetradexocylphosphonio]-2-hydroxypropane-1-phosphate;
3-[N,N-dipropyl-N-3-dodecoxy-2-hydroxypropylammonio]-propane-1-phosphonate;
3-(N,N-dimethyl-N-hexadecylammonio)propane-1-sulfonate;
3-(N,N-dimethyl-N-hexadecylammonio)-2-hydroxypropane-1-sulfonate;
4-[N,N-di(2-hydroxypethyl)-N-(2-hydroxydodecyl)ammonio]butane-1-carboxylate;
3-[S-ethyl-S-(3-dodecoxy-2-hydroxypropyl)sulfonio]- propane-1-phosphate;
3-[P,P-dimethyl-P-dodecylphosphonio]-propane-1-phosphonate; and
5-[N,N-di(3-hydroxypropyl)-N-hexadecylammonio]-2-hydroxy-pentane-1-sulfate.

Amphoteric detergents which may be used in this invention include at least one acid group. This may be a carboxylic or a sulphonic acid group. They include quaternary nitrogen and therefore are quaternary amido acids. They should generally include an alkyl or alkenyl group of 7 to 18 carbon atoms. They will usually comply with an overall structural formula: 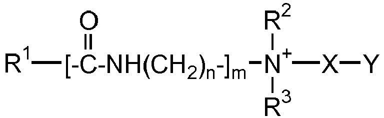
where R¹ is alkyl or alkenyl of 7 to 18 carbon atoms;
R² and R³ are each independently alkyl, hydroxyalkyl or carboxyalkyl of 1 to 3 carbon atoms;
n is 2 to 4;
m is 0 to 1;
X is alkylene of 1 to 3 carbon atoms optionally substituted with hydroxyl, and
Y is -CO₂ or -SO₃⁻.

Suitable amphoteric detergents within the above general formula include simple betaines of formula: 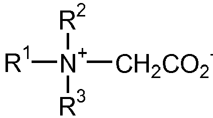 and amido betaines of formula: 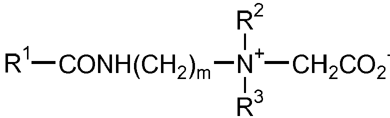 where m is 2 or 3, preferably 3.

In both formulae R¹, R² and R³ are as defined previously. R¹ may in particular be a mixture of C₁₂ and C₁₄ alkyl groups derived from coconut so that at least half, preferably at least three quarters of the groups R¹ have 10 to 14 carbon atoms. R² and R³ are preferably methyl. Alkylamidopropyl betaines are preferred, for example Cocamidopropyl Betaine.

A further possibility is that the amphoteric detergent is a sulphobetaine of formula: 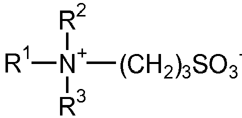 or 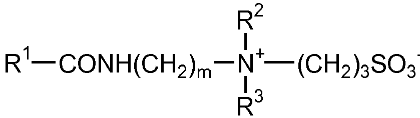 where m is 2 or 3, or variants of these in which -(CH₂)₃SO₃⁻ is replaced by

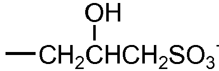

In these formulae R¹, R² and R³ are as discussed previously.

Amphoacetates and amphodiacetates, for example cocoamphoacetate and cocoamphodiacetate, may also be used as zwitterionic and/or amphoteric compounds in the present invention. Amphoacetates and amphodiacetates may be described as fatty alkyl imidazolines obtained by the condensation of fatty acids or their esters with aminoethylethanol amine.

The preferred amphoteric/zwitterionic surfactants for use in the present cleansing composition are alkylamidopropyl betaines, such as Cocamidopropyl Betaine, and amphoacetates and amphodiacetates, the alkylamidopropyl betaines being most preferred.
Typically, the amphoteric/zwitterionic surfactant(s), including the preferred ones described above, comprise(s) 0.1 to 20 % by weight, preferably 0.5 to 15 % by weight, more preferably 1 to 10 % by weight, even more preferably 1 to 8 and most preferably 1.5 to 5 % by weight of the total cleansing composition.

### (iii) Nonionic surfactants

In addition to one or more anionic and amphoteric and/or zwitterionic surfactants, the surfactant system may optionally comprise a nonionic surfactant or a mixtrure of at least two non-ionic surfactants.

The nonionic surfactant which may be used includes in particular the reaction products of compounds having a hydrophobic group and a reactive hydrogen atom, for example aliphatic alcohols, acids, amides or alkyl phenols with alkylene oxides, especially ethylene oxide either alone or with propylene oxide. Specific nonionic detergent compounds are (C₆-C₂₂) alkyl phenols-ethylene oxide condensates, the condensation products of aliphatic primary or secondary linear or branched (C₈-C₁₈) alcohols with ethylene oxide, and products made by condensation of ethylene oxide with the reaction products of propylene oxide and ethylenediamine. Other so-called nonionic detergent compounds include long chain tertiary amine oxides, long chain tertiary phosphine oxides and dialkyl sulphoxides.

The nonionic surfactant may also be a sugar amide, such as a polysaccharide amide. Specifically, the surfactant may be one of the lactobionamides described in U.S. Patent No. 5,389,279 or it may be one of the sugar amides described in Patent No. 5,009,814.

Other non-ionic surfactants which may be used are described in U.S. Patent No. 3,723,325 and alkyl polysaccharide nonionic surfactants as disclosed in U.S. Patent No. 4,565,647.

Preferred alkyl polysaccharides are alkylpolyglycosides of the formula

R²O(CₙH₂ₙO)ₜ(glycosyl)ₓ

wherein R² is selected from the group consisting of alkyl, alkylphenyl, hydroxyalkyl, hydroxyalkylphenyl, and mixtures thereof in which alkyl groups contain from about 10 to about 18, preferably from about 12 to about 14, carbon atoms; n is 0 to 3, preferably 2; t is from 0 to about 10, preferably 0; and x is from 1.3 to about 10, preferably from 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the I-position). The additional glycosyl units can then be attached between their 1-position and the preceding glycosyl units 2-, 3-, 4- and/or 6-position, preferably predominantly the 2-position.

Nonionic surfactant(s) comprise(s) 0 to 10 % by weight of the cleansing composition. When present, they preferably comprise 1 to 10 % by weight, more preferably 2 to 10 % by weight of the cleansing composition.

In certain embodiments the personal cleansing compositions may include optional ingredients such as those common in the art as ingredients in personal cleansing composition. Typically additional ingredients include thickening agents, humectants (moisturizers), conditioners, antioxidants, preservatives (antimicrobials), fragrances, benefit agents, colorants (generally in low amounts from 0.0001 to 0.01 % by weight based on the total weight of the cleansing composition), pearlescent agents (e.g. glycol distearate and pearlescent pigments), organic acids (e.g. citric acid or tartaric acid), and sequestering agents (e.g. Disodium EDTA (disodium dihydrogen ethylenediaminetetraacetate).

Optionally, the personal cleansing composition of the present invention may comprise a further thickening agent (in addition to the hydrophobically modified crosslinked acrylic copolymer) such as xanthan gum, hydroxypropyl methylcellulose, fatty acid alkanolamides (e.g. Cocamide MEA) and mixtures thereof, xanthan gum being preferred. When used the additional thickening agent(s), including the preferred ones, is (are) typically employed in a total amount from 0.01 to 5 % by weight, preferably from 0.1 to 3.0 % by weight, more preferably from 0.2 to 1.5 % by weight, and most preferably from 0.3 to 1 % by weight, each based on the total weight of the cleansing composition.
Examples of humectants (moisturizers) for optional use in the personal cleansing composition are glycerol, sorbitol, propylene glycol, amino acids (e.g. glycine, alanine), pyrrolidone carboxylic acid (pidolic acid (PCA)), and mixtures thereof, glycerol being preferred. When used the humectant(s), including the preferred ones, is (are) typically employed in a total amount from 0.05 to 10 % by weight, preferably from 0.1 to 8 % by weight, more preferably from 1 to 7 % by weight, and most preferably from 2.5 to 5.5 % by weight, each based on the total weight of the cleansing composition.

Conditioners may also be included in the personal cleansing composition of the present invention. Preferred conditioners are proteins and protein derivatives such as collagen and silk hydrolysates and milk proteins, as well as cationic conditioners such as Lauryl Methyl Gluceth-10 Hydroxypropyldimonium Chloride, and polycationic polymers, preferably those that are known under the INCI name Polyquaternium. Polyquaternium is a neologism used to emphasize the presence of quaternary ammonium groups in the polymer. Their positive charges ionically bond them to hair and skin. Examples of particular suitable Polyquaternium polymers include Polyquaternium-7 (copolymer of acrylamide and diallyldimethylammonium chloride) and Polyquaternium-10 (quaternized hydroxyethylcellulose). Polyquaternium polymers, especially Polyquaternium-7, are preferred. Mixtures of at least two different conditioners may also be employed. When used the conditioner(s), including the preferred ones, is (are) typically employed in a total amount from 0.01 to 5 % by weight based on the total weight of the cleansing composition. When polycationic polymer(s) such as Polyquaternium polymer(s) is (are) used the total amount preferably ranges from 0.005 to 1 % by weight, more preferably from 0.01 to 0.5 % by weight, even more preferably from 0.01 to 0.1 % by weight, and most preferably from 0.02 to 0.05 % by weight, each based on the total weight of the cleansing composition.

Antioxidants may optionally be added to protect the personal cleansing composition against the degradation by oxygen. Typical antioxidants include tocopherols, e.g. α-tocopherol, BHT (butylated hydroxytoluene), BHA (butylated hydroxyanisole), propylgallate, t-butylhydroquinone, and ascorbyl palmitate. Mixtures of at least two different antioxidants may also be employed. When used the antioxidant(s), including the preferred ones, is (are) typically employed in a total amount from 0.001 to 5 % by weight, preferably from 0.005 to 1 % by weight, more preferably from 0.01 to 0.5 % by weight, and most preferably from 0.05 to 0.2 % by weight, each based on the total weight of the cleansing composition. Preservatives (antimicrobials) may optionally be added to protect the personal cleansing composition against the degradation by microorganisms. Typical preservatives include:
- aromatic alcohols such as phenoxyethanol, benzyl alcohol, phenethyl alcohol, phenoxyisopropanol;
- parabens such as methylparaben, ethylparaben, butylparaben, propylparaben; and isobutylparaben;
- aldehydes such as formaldehyde, paraformaldehyde, and glutaraldehyde;
- 1,2-alkandiols having 5 to 22 carbons in the carbon chain such as 1,2-pentandiol, 1,2-hexandiol, 1,2-heptandiol, 1,2-decandiol, 1,2-dodecandiol, and 1,2-hexadecandiol;
- formaldehyde releasing compounds such as DMDM hydantoin and diazolidinyl urea;
- halogenated compounds such as isothiazolinones, e.g.
   methylchloroisothiazolinone and methylisothiazolinone, triclosan, triclocarban, iodopropynylbutylcarbamat, 5-bromo-5-nitro-1,3-dioxan, chlorhexidindigluconate and chlorhexidin acetate, 2-bromo-2-nitropropane-1,3-diol, methyldibromoglutaronitrile;
- inorganic compounds such as sulfites, boric acid und borates, bisulfites;
- cationic compounds such as Quaternium-15, benzalkonium chloride, benzethonium chloride, polyaminopropylbiguanide;
- organic acids and their physiologically acceptable salts such as citric acid, lactic acid, acetic acid, benzoe acid, sorbic acid e.g. sodium and potassium sorbate, salicylic acid, dehydroacetic acid.

Mixtures of at least two different preservatives may also be employed. The amount of preservative(s) to be used in the present invention strongly depends on the type of preservative. If sorbates, e.g. a combination of sodium and potassium sorbate, are used they are typically employed in a total amount of from 0.2 to 1.5 % by weight, preferably from 0.3 to 1.0 % by weight, and more preferably from 0.4 to 0.8 % by weight, each based on the total weight of the cleansing composition. The personal cleansing composition of the present invention may optionally contain further benefit agents such as nonfat dry milk, plant extracts, e.g. aqueous extracts, alcoholic extracts, aqueous-alcoholic extracts and glycolic extracts. When used nonfat dry milk is typically employed in an amount of from 0.01 to 3 % by weight, preferably from 0.05 to 2 % by weight, more preferably from 0.1 to 1 % by weight, and most preferably from 0.2 to 0.6 % by weight, each based on the total weight of the cleansing composition.

Ester oils may also be included in the personal cleansing composition of the present invention; however there is no necessity to incorporate them into the present formulations because the stability of the formulations and the beneficial effect to the skin are already ensured without the addition of ester oils. Ester oils are synthetic lipids which are prepared by first cleaving the triglycerides of vegetable oils into fatty acids and glycerol and then esterifying the fatty acids selectively with glycerol, saccharose, glycose or other alcohols to obtain a new and intended combination of fatty acid and alcohol. The term "ester oils" also includes esters that are alkoxylated at the alcohol or ester part or both, e.g. PEG-7 Glycerol Cocoate. An extensive description of ester oils including various specific examples can be found in DE 10 2008 037 633 A1. It is preferred that the present cleansing compositions are free of any synthetic ester oils. For example the cleansing compositions are preferably free of PEG-7 Glycerol Cocoate.

Generally, the personal cleansing composition of the present invention is an oil-in-water emulsion. Water makes up the remainder of the cleansing composition. It is typically comprised in the cleansing composition in amounts of from 5 to 94.97 % by weight, preferably from 20 to 94 % by weight, more preferably from 30 to 90 % by weight, even more preferably from 50 to 90 % by weight, still more preferably from 55 to 87 % by weight, and most preferably from 65 to 85 % by weight, each based on the total weight of the cleansing composition.

The emulsions of the present invention are stable over the time, even at higher temperatures such as about 40°C and at changing temperatures. The emulsions may have a clear or opaque appearance, typically they are opaque.

In certain embodiments of the present invention the personal cleansing composition comprises:
(a) 0.02 to 10 % by weight, preferably 0.03 to 5 % by weight, more preferably 0.05 to 3 % by weight of the nourishing oil component;
(b) 0.5 to 10 % by weight, preferably 1 to 8 % by weight, more preferably 2 to 5.5 % by weight of the compatibilizing oil component;
(c)(i) 5 to 25 % by weight, preferably 5 to 20 % by weight, more preferably 6 to 15 % by weight, most preferably 7 to 12 % by weight of anionic surfactant or mixture of anionic surfactants;
(c)(ii) 0.5 to 15 % by weight, preferably 1 to 10 % by weight, more preferably 1 to 8 % by weight, most preferably 1.5 to 5 % by weight of amphoteric and/or zwitterionic surfactant or mixtures thereof;
(d) 0.05 to 4 % by weight, preferably 0.1 to 3 % by weight, more preferably 0.5 to 2 % by weight, even more preferably 0.6 to 1.5 % by weight, most preferably 0.7 to 1.3 % by weight of hydrophobically modified crosslinked acrylic copolymer; and
(e) water, and
(f) 0.05 to 10 % by weight, preferably 0.1 to 8 % by weight, more preferably 1 to 7 % by weight, and most preferably 2.5 to 5.5 % by weight of humectant(s),
wherein all weight percentages of the components are based on the total weight of the cleansing composition and water is present in an amount adding up to 100 % by weight; typically in an amount of from 20 to 93.88 % by weight, preferably from 35 to 92.77 % by weight, more preferably from 55 to 90.47 % by weight, and most preferably from 65 to 86.25 % by weight.

The pH value of the personal cleansing composition of the present invention is typically within the range of from 4 to 7, preferably from 4.5 to 6.5, more preferably from 4.5 to 6, and most preferably from 4.8 to 5.5.

Typically, the present personal cleansing composition is in gel form. In preferred embodiments the viscosity of the cleansing composition is within the range of from 3,000 to 10,000 mPa·s and more preferred from 5,000 to 10,000. The viscosity is measured immediately after preparation of the cleansing composition with a Brookfiled DV-II+ Viscosimeter, Model LVDV-II+, spindle 4 at 60 rpm and 20°C

The present personal cleansing composition may be used to clean the skin/and or hair, e.g. as shower gels, facial cleaners, liquid hand soaps, and shampoos. Preferably, they are used as shower gels.

Due to the presence of the oil components the application of the present cleansing composition to the skin does not only provide a cleaning effect but also balances the lipid content of the skin. The user feels pampered with the these formulations that soothe the skin and leave the skin feeling silky-smooth.

The invention will be illustrated by the following examples.

### EXAMPLES

All percentages are given in weight percent.

**Specification of ingredients used:**

| | |
|---|---|
| Carbopol® Ultrez 20: | ACRYLATES/C10-30 ALKYL ACRYLATE CROSSPOLYMER (available from Lubrizol Advanced Materials, Inc.) |
| Carbopol® EDT 2020: | : ACRYLATES/C10-30 ALKYL ACRYLATE CROSSPOLYMER (available from Lubrizol Advanced Materials, Inc.) |
| Keltrol CG-SFT: | XANTHAN GUM (available from RAHN GmbH, Frankfurt, Germany) |
| Ethersulfate-Na 70 %: | SODIUM LAURETH SULFATE 70 %, AQUA 30 % |
| Caustic soda 50 %: | SODIUM HYDROXIDE 50 %, AQUA 50 % |
| Betaine 40 % | AQUA 53.20 %, COCAMIDOPROPYL BETAINE 40.00 %, SODIUM CHLORIDE 6.80 % |
| Polyquaternium 7 (8.5 %): | %): AQUA 91.38 %, POLYQUATERNIUM-7 8.50 %, METHYLPARABEN 0.10 %, PROPYLPARABEN 0.02 % |
| Glycerol 99.5 %: | GLYCERIN 99.50 %, AQUA 0.50 % |
| Preservative 40 %: | AQUA 60,00 %, SODIUM BENZOATE 33,33 %, POTASSIUM SORBATE 6,67 % |
| Tocopherol 70 %: | TOCOPHEROL 70 %, HELIANTHUS ANNUUS (SUNFLOWER) SEED OIL 30 % |
| OPTIPHEN MIT plus: | PHENETHYL ALCOHOL 45 %, PPG-2 METHYL ETHER 32 %, AQUA 18 %, |
| | METHYLISOTHIAZOLINONE 5 % (available from ISP BIOCHEMA, Memmingen, Germany) |
| ROKONSAL PB-4: | PHENOXYETHANOL 72.00 %, METHYLPARABEN 14.50 %, ETHYLPARABEN 5.80 %, BUTYLPARABEN 5.30 %, PROPYLPARABEN 2.40 % (available from ISP BIOCHEMA, Memmingen, Germany) |
| Marula oil: | 100 %, pressed (available from S&D Chesham, Bergisch Gladbach, Germany) |
| Soybean oil: | 100 %, refined (available from Henry Lamotte Oils GmbH, Bremen, Germany) |
| Sunflower oil stab.: | HELIANTHUS ANNUS SEED OIL 99.995 %, CITRIC ACID 0.005 %, refined (available from Henry Lamotte Oils GmbH, Bremen, Germany) |
| Coconut oil: | 100 %, refined (available from Henry Lamotte Oils GmbH, Bremen, Germany) |

As indicated above sunflower oil is used as stabilized oils. All other vegetable oils are used as pure oils.

Examples 1 to 6 describe the compositions of shower gels. They were prepared by first charging the water into a vessel and then adding Carbopol® polymer. When the Carbopol® polymer was sunken stirring was started and Ethersulfate-Na 70 % was added. Afterwards, the pH-value was adjusted to 4.3 with caustic soda 50 %, and then Betaine 40 % and the remaining ingredients were added. In the examples containing dry milk the milk powder was predispersed in the compatibilizing oil and then added together with Betaine 40 % and the remaining ingredients.

**Example 1**

| **Ingredient** | **Amount/kg** |
|---|---|
| Water | 66.565 |
| Carbopol® Ultrez 20 | 0.900 |
| Ethersulfate-Na 70 % | 13.600 |
| Caustic soda 50 % | 0.110 |
| Betaine 40 % | 8.000 |
| Nonfat Dry Milk (SINE ADIPE LAC) | 0.375 |
| Polyquaternium 7 (8.5 %) | 0.300 |
| Glycerol 99.5 % | 3.000 |
| Preservative 40 % | 1.500 |
| Tocopherol 70 % | 0.150 |
| Fragrance | 0.500 |
| Soybean oil | 4.950 |
| Marula oil | 0.050 |
| | **100.000** |

**Example 2**

| **Ingredient** | **Amount/kg** |
|---|---|
| Water | 66.565 |
| Carbopol® Ultrez 20 | 0.900 |
| Ethersulfate-Na 70 % | 13.600 |
| Caustic soda 50 % | 0.110 |
| Betaine 40 % | 8.000 |
| Nonfat Dry Milk (SINE ADIPE LAC) | 0.375 |
| Polyquaternium 7 (8.5 %) | 0.300 |
| Glycerol 99.5 % | 3.000 |
| Preservative 40 % | 1.500 |
| Tocopherol 70 % | 0.150 |
| Fragrance | 0.500 |
| Soybean oil | 2.500 |
| Marula oil | 2.500 |
| | **100.000** |

**Example 3**

| **Ingredient** | **Amount/kg** |
|---|---|
| Water | 66.465 |
| Carbopol® Ultrez 20 | 1.000 |
| Ethersulfate-Na 70 % | 13.600 |
| Caustic soda 50 % | 0.110 |
| Betaine 40 % | 8.000 |
| Nonfat Dry Milk (SINE ADIPE LAC) | 0.375 |
| Polyquaternium 7 (8.5 %) | 0.300 |
| Glycerol 99.5 % | 3.000 |
| Preservative 40 % | 1.500 |
| Tocopherol 70 % | 0.150 |
| Fragrance | 0.500 |
| Soybean oil | 4.950 |
| Marula oil | 0.050 |
| | **100.000** |

**Example 4**

| **Ingredient** | **Amount/kg** |
|---|---|
| Water | 63.825 |
| Carbopol® EDT 2020 | 0.900 |
| Keltrol CG-SFT | 0.500 |
| Ethersulfate-Na 70 % | 13.600 |
| Caustic soda 50 % | 0.600 |
| Betaine 40 % | 8.000 |
| Nonfat Dry Milk (SINE ADIPE LAC) | 0.375 |
| Polyquaternium 7 (8.5 %) | 0.300 |
| Glycerol 99.5 % | 5.000 |
| OPTIPHEN MIT PLUS | 0.200 |
| ROKONSAL PB-4 | 1.000 |
| Fragrance | 0.700 |
| Soybean oil | 4.900 |
| Marula oil | 0.100 |
| | **100.000** |

**Example 5**

| **Ingredient** | **Amount/kg** |
|---|---|
| Water | 64.825 |
| Carbopol® Ultrez 20 | 0.800 |
| Ethersulfate-Na 70 % | 13.600 |
| Caustic soda 50 % | 0.600 |
| Betaine 40 % | 8.000 |
| Nonfat Dry Milk (SINE ADIPE LAC) | 0.375 |
| Polyquaternium 7 (8.5 %) | 0.300 |
| Glycerol 99.5 % | 5.000 |
| Preservative 40 % | 1.500 |
| Coconut oil refined | 4.900 |
| Marula oil | 0.100 |
| | **100.000** |

**Example 6**

| **Ingredient** | **Amount/kg** |
|---|---|
| Water | 64.825 |
| Carbopol® Ultrez 20 | 0.800 |
| Ethersulfate-Na 70 % | 13.600 |
| Caustic soda 50 % | 0.600 |
| Betaine 40 % | 8.000 |
| Nonfat Dry Milk (SINE ADIPE LAC) | 0.375 |
| Polyquaternium 7 (8.5 %) | 0.300 |
| Glycerol 99.5 % | 5.000 |
| Preservative 40 % | 1.500 |
| Sunflower oil stab. | 4.900 |
| Marula oil | 0.100 |
| | **100.000** |

All shower gels of Examples 1 to 6 are oil-in-water emulsions having an opaque, white appearance. The emulsions are stable at -10°C, 5°C, 20°C and 40°C for at least 3 months. They are also stable for at least 3 months when subjected to continuous temperature fluctuations (heating/cooling cycle: from -10°C to 40°C within 24 h and from 40°C to -10° within 24 h).

## Claims

1. A personal cleansing composition comprising:
(a) 0.01 to 15 % by weight of a nourishing oil component which is a vegetable oil having an oleic acid content of at least 38 % by weight (based on the total amount of esterified fatty acids in the vegetable oil) and which is marula oil, wherein when one or more of apricot kernel oil, argan oil and tamanu oil are present the total amount of marula oil, apricot kernel oil, argan oil, and tamanu oil is 0.01 to 15 % by weight;
(b) 0.01 to 20 % by weight of a compatibilizing oil component which is a vegetable oil or mixture of vegetable oils being selected from soybean oil, olive oil, coconut oil, and sunflower oil;
wherein the total amount of nourishing oil component and compatibilizing oil component does not exceed 25 % by weight;
(c) 5 to 50 % by weight of a surfactant system comprising:
(i) an anionic surfactant or mixture of anionic surfactants and
(ii) an amphoteric and/or zwitterionic surfactant or mixtures thereof;
(d) 0.01 to 5.0 % by weight of hydrophobically modified crosslinked acrylic copolymer; and
(e) water,
wherein all weight percentages of the components are based on the total weight of the cleansing composition and water is present in an amount adding up to 100 % by weight.

2. The personal cleansing composition of claim 1 wherein the hydrophobically modified crosslinked acrylic copolymer is a crosslinked copolymer of (i) acrylic acid and/or methacrylic acid, and (ii) alkyl acrylates and/or alkyl methacrylates.

3. The personal cleansing composition of claim 3 wherein the hydrophobically modified crosslinked acrylic copolymer is a crosslinked polymer of acrylic acid, C₁₀-C₃₀ alkyl acrylate and C₁₀-C₃₀ alkyl methacrylate.

4. The personal cleansing composition of any of the preceding claims wherein the anionic surfactant is selected from alkyl sulfates, alkyl ether sulfates, succinates, acyl glutamates and mixtures thereof.

5. The personal cleansing composition of any of the preceding claims wherein the amphoteric and/or zwitterionic surfactant is selected from alkylamidopropyl betaines, amphoacetates, amphodiacetates, and mixtures thereof.

6. The personal cleansing composition of any of the preceding claims comprising:
(c)(i) 4.9 to 30 % by weight of an anionic surfactant or mixture of anionic surfactants; and
(c)(ii) 0.1 to 20 % by weight an amphoteric and/or zwitterionic surfactant or mixtures thereof;
each based on the total weight of the cleansing composition.

7. The personal cleansing composition of any of the preceding claims wherein the weight ratio of nourishing oil component (a) to compatibilizing oil component (b) ranges from 1:700 to 10:1.

8. The personal cleansing composition of any of the preceding claims further comprising (f) an humectant, preferably in an amount of from 0.05 to 10 % by weight based on the total weight of the cleansing composition.

9. The personal cleansing composition of claim 8 wherein the humectant is glycerol.

10. The personal cleansing composition of any of the preceding claims further comprising (g) a conditioner, preferably in an amount of from 0.01 to 5 % by weight based on the total weight of the cleansing composition.

11. The personal cleansing composition of claim 10 wherein the conditioner is a cationic conditioner, preferably a polycationic polymer.

12. The personal cleansing composition of any of the preceding claims comprising:
(a) 0.02 to 10 % by weight, preferably 0.03 to 5 % by weight of the nourishing oil component;
(b) 0.5 to 10 % by weight, preferably 1 to 8 % by weight of the compatibilizing oil component;
(c)(i) 5 to 25 % by weight, preferably 6 to 15 % by weight of anionic surfactant or mixture of anionic surfactants;
(c)(ii) 0.5 to 15 % by weight, preferably 1 to 8 % by weight of amphoteric and/or zwitterionic surfactant or mixtures thereof;
(d) 0.1 to 3 % by weight, preferably 0.6 to 1.5 % by weight of hydrophobically modified crosslinked acrylic copolymer;
(e) water, and
(f) 0.1 to 8 % by weight, preferably 1 to 7 % by weight of humectant(s), wherein all weight percentages of the components are based on the total weight of the cleansing composition and water is present in an amount adding up to 100 % by weight.

13. The personal cleansing composition of any of the preceding claims further comprising an additional thickening agent different from the hydrophobically modified crosslinked acrylic polymer (d).

14. The personal cleansing composition of any of the preceding claims which is free of any synthetic ester oil.

15. Use of the personal cleansing composition of any of the preceding claims to clean the skin and/or hair.

## Patentansprüche

1. Körperreinigungszusammensetzung, enthaltend:
(a) 0,01 bis 15 Gew.-% einer nährenden Ölkomponente, die ein Pflanzenöl mit einem Ölsäuregehalt von mindestens 38 Gew.-% (bezogen auf die Gesamtmenge von veresterten Fettsäuren in dem Pflanzenöl) ist und die Marulaöl ist, wobei, wenn ein oder mehrere von Aprikosenkernöl, Arganöl und Tamanuöl vorhanden sind, die Gesamtmenge von Marulaöl, Aprikosenkernöl, Arganöl und Tamanuöl 0,01 bis 15 Gew.-% beträgt.
(b) 0,01 bis 20 Gew.-% einer kompatibilisierenden Ölkomponente, die ein Pflanzenöl oder eine Mischung von Pflanzenölen ist, ausgewählt aus Sojaöl, Olivenöl, Kokosöl und Sonnenblumenöl,
wobei die Gesamtmenge von nährender Ölkomponente und kompatibilisierender Ölkomponente 25 Gew.-% nicht übersteigt,
(c) 5 bis 50 Gew.-% eines Tensidsystems, enthaltend:
(i) ein anionisches Tensid oder eine Mischung von anionischen Tensiden und
(ii) ein amphoteres und/oder zwitterionisches Tensid oder Mischungen davon,
(d) 0,01 bis 5,0 Gew.-% eines hydrophob modifizierten vernetzten Acrylcopolymers und
(e) Wasser,
wobei alle Gewichtsprozentangaben der Komponenten auf dem Gesamtgewicht der Reinigungszusammensetzung basieren und Wasser in einer Menge vorhanden ist, die sich zu 100 Gew.-% addiert.

2. Körperreinigungszusammensetzung nach Anspruch 1, wobei das hydrophob modifizierte vernetzte Acrylcopolymer ein vernetztes Copolymer von (i) Acrylsäure und/oder Methacrylsäure und (ii) Alkylacrylaten und/oder Alkylmethacrylaten ist.

3. Körperreinigungszusammensetzung nach Anspruch 3, wobei das hydrophob modifizierte vernetzte Acrylcopolymer ein vernetztes Polymer von Acrylsäure, C₁₀-C₃₀-Alkylacrylat und C₁₀-C₃₀-Alkylmethacrylat ist.

4. Körperreinigungszusammensetzung nach einem der vorstehenden Ansprüche, wobei das anionische Tensid aus Alkylsulfaten, Alkylethersulfaten, Succinaten, Acylglutamaten und Mischungen davon ausgewählt ist.

5. Körperreinigungszusammensetzung nach einem der vorstehenden Ansprüche, wobei das amphotere und/oder zwitterionische Tensid aus Alkylamidopropylbetainen, Amphoacetaten, Amphodiacetaten und Mischungen davon ausgewählt ist.

6. Körperreinigungszusammensetzung nach einem der vorstehenden Ansprüche, enthaltend:
(c)(i) 4,9 bis 30 Gew.-% eines anionischen Tensids oder einer Mischung von anionischen Tensiden und
(c)(ii) 0.1 bis 20 Gew.-% eines amphoteren und/oder zwitterionischen Tensids oder Mischungen davon,
jeweils bezogen auf das Gesamtgewicht der Reinigungszusammensetzung.

7. Körperreinigungszusammensetzung nach einem der vorstehenden Ansprüche, wobei das Gewichtsverhältnis von nährender Ölkomponente (a) zu kompatibilisierender Ölkomponente (b) von 1:700 bis 10:1 reicht.

8. Körperreinigungszusammensetzung nach einem der vorstehenden Ansprüche, die ferner (f) ein Feuchthaltemittel, vorzugsweise in einer Menge von 0,05 bis 10 Gew.-%, bezogen auf das Gesamtgewicht der Reinigungszusammensetzung, enthält.

9. Körperreinigungszusammensetzung nach Anspruch 8, wobei das Feuchthaltemittel Glycerin ist.

10. Körperreinigungszusammensetzung nach einem der vorstehenden Ansprüche, die ferner (g) ein Konditionierungsmittel, vorzugsweise in einer Menge von 0,01 bis 5 Gew.-%, bezogen auf das Gesamtgewicht der Reinigungszusammensetzung, enthält.

11. Körperreinigungszusammensetzung nach Anspruch 10, wobei das Konditionierungsmittel ein kationisches Konditionierungsmittel, vorzugsweise ein polykationisches Polymer ist.

12. Körperreinigungszusammensetzung nach einem der vorstehenden Ansprüche enthaltend:
(a) 0,02 bis 10 Gew.-%, vorzugsweise 0,03 bis 5 Gew.-% der nährenden Ölkomponente,
(b) 0,5 bis 10 Gew.-%, vorzugsweise 1 bis 8 Gew.-% der kompatibilisierenden Ölkomponente,
(c)(i) 5 bis 25 Gew.-%, vorzugsweise 6 bis 15 Gew.-% anionisches Tensid oder Mischungen von anionischen Tensiden,
(c)(ii) 0.5 bis 15 Gew.-%, vorzugsweise 1 bis 8 Gew.-% amphoteres und/oder zwitterionisches Tensid oder Mischungen davon,
(d) 0,1 bis 3 Gew.-%, vorzugsweise 0,6 bis 5 Gew.-% hydrophob modifiziertes vernetztes Acrylcopolymer,
(e) Wasser und
(f) 0,1 bis 8 Gew.-%, vorzugsweise 1 bis 7 Gew.-% Feuchthaltemittel, wobei alle Prozentangaben der Komponenten auf das Gesamtgewicht der Reinigungszusammensetzung bezogen sind und Wasser in einer Menge vorhanden ist, die sich zu 100 Gew.-% addiert.

13. Körperreinigungszusammensetzung nach einem der vorstehenden Ansprüche, die ferner ein zusätzliches Verdickungsmittel, das sich von dem hydrophob modifizierten vernetzten Acrylpolymer (d) unterscheidet, enthält.

14. Körperreinigungszusammensetzung nach einem der vorstehenden Ansprüche, die frei von irgendeinem synthetischen Esteröl ist.

15. Verwendung der Körperreinigungszusammensetzung nach einem der vorstehenden Ansprüche, um die Haut oder Haare zu reinigen.

## Revendications

1. Composition de soin personnel de nettoyage, comprenant :
a) de 0,01 à 15 % en poids d'une composante huile nourrissante qui est une huile végétale dont la teneur en acide oléique vaut au moins 38 % en poids, par rapport à la quantité totale d'acides gras estérifiés dans cette huile végétale, et qui est de l'huile de marula, étant entendu que, lorsqu'il y a de l'une ou de plusieurs des huiles d'amande d'abricot, d'argan et de tamanu, la proportion totale d'huile de marula, d'huile d'amande d'abricot, d'huile d'argan et d'huile de tamanu vaut de 0,01 à 15 % en poids,
b) de 0,01 à 20 % en poids d'une composante huile compatibilisante qui est une huile végétale ou un mélange d'huiles végétales, choisie(s) parmi de l'huile de soja, de l'huile d'olive, de l'huile de noix de coco et de l'huile de tournesol,
étant entendu que la proportion totale de composante huile nourrissante et de composante huile compatibilisante n'excède pas 25 % en poids,
c) de 5 à 50 % en poids d'un système tensioactif comportant :
i) un tensioactif anionique ou un mélange de tensioactifs anioniques,
ii) et un tensioactif amphotère et/ou zwitterionique ou un mélange de tels tensioactifs,
d) de 0,01 à 5,0 % en poids d'un copolymère polyacrylique réticulé, modifié de manière à être hydrophobe,
e) et de l'eau ;
étant entendu que les pourcentages pondéraux de tous les composants sont rapportés au poids total de la composition de nettoyage et que l'eau s'y trouve en une quantité assurant le complément à 100 % en poids.

2. Composition de soin personnel de nettoyage, conforme à la revendication 1, dans laquelle le copolymère polyacrylique réticulé, modifié de manière à être hydrophobe, est un copolymère réticulé
i) d'acide acrylique et/ou d'acide méthacrylique,
ii) et d'acrylate(s) d'alkyle et/ou de méthacrylate(s) d'alkyle.

3. Composition de soin personnel de nettoyage, conforme à la revendication 3, dans laquelle le copolymère polyacrylique réticulé, modifié de manière à être hydrophobe, est un polymère réticulé d'acide acrylique, d'acrylate d'alkyle en C₁₀-C₃₀ et de méthacrylate d'alkyle en C₁₀-C₃₀.

4. Composition de soin personnel de nettoyage, conforme à l'une des revendications précédentes, dans laquelle le tensioactif anionique est choisi parmi les alkyl-sulfates, les alkyl-éther-sulfates, les succinates, les acyl-glutamates et leurs mélanges.

5. Composition de soin personnel de nettoyage, conforme à l'une des revendications précédentes, dans laquelle le tensioactif amphotère et/ou zwitterionique est choisi parmi les alkyl-amido-propyl-bétaïnes, les ampho-acétates, les ampho-diacétates et leurs mélanges.

6. Composition de soin personnel de nettoyage, conforme à l'une des revendications précédentes, comprenant :
c) i) de 4,9 à 30 % en poids d'un tensioactif anionique ou d'un mélange de tensioactifs anioniques,
c) ii) et de 0,1 à 20 % en poids d'un tensioactif amphotère et/ou zwitterionique ou d'un mélange de tels tensioactifs,
chacun de ces pourcentages étant rapporté au poids total de la composition de nettoyage.

7. Composition de soin personnel de nettoyage, conforme à l'une des revendications précédentes, dans laquelle le rapport pondéral de la composante huile nourrissante (a) à la composante huile compatibilisante (b) vaut de 1/700 à 10/1.

8. Composition de soin personnel de nettoyage, conforme à l'une des revendications précédentes, comprenant en outre f) un agent humectant,
de préférence présent en une proportion de 0,05 à 10 %, en poids rapporté au poids total de la composition de nettoyage.

9. Composition de soin personnel de nettoyage, conforme à la revendication 8, dans laquelle l'agent humectant est du glycérol.

10. Composition de soin personnel de nettoyage, conforme à l'une des revendications précédentes, comprenant en outre g) un agent conditionneur,
de préférence présent en une proportion de 0,01 à 5 %, en poids rapporté au poids total de la composition de nettoyage.

11. Composition de soin personnel de nettoyage, conforme à la revendication 10, dans laquelle l'agent conditionneur est un agent conditionneur cationique, de préférence un polymère polycationique.

12. Composition de soin personnel de nettoyage, conforme à l'une des revendications précédentes, comprenant :
a) de 0,02 à 10 % en poids, et de préférence de 0,03 à 5 % en poids, de la composante huile nourrissante,
b) de 0,5 à 10 % en poids, et de préférence de 1 à 8 % en poids, de la composante huile compatibilisante,
c) i) de 5 à 25 % en poids, et de préférence de 6 à 15 % en poids, d'un tensioactif anionique ou d'un mélange de tensioactifs anioniques,
c) ii) de 0,5 à 15 % en poids, et de préférence de 1 à 8 % en poids, d'un tensioactif amphotère et/ou zwitterionique ou d'un mélange de tels tensioactifs,
d) de 0,1 à 3 % en poids, et de préférence de 0,6 à 1,5 % en poids, d'un copolymère polyacrylique réticulé, modifié de manière à être hydrophobe,
e) de l'eau ;
f) et de 0,1 à 8 % en poids, et de préférence de 1 à 7 % en poids, d'un agent ou d'agents humactant(s) ;
étant entendu que les pourcentages pondéraux de tous les composants sont rapportés au poids total de la composition de nettoyage et que l'eau s'y trouve en une quantité assurant le complément à 100 % en poids.

13. Composition de soin personnel de nettoyage, conforme à l'une des revendications précédentes, qui comprend en outre un agent épaississant supplémentaire, différent du copolymère polyacrylique réticulé, modifié de manière à être hydrophobe (d).

14. Composition de soin personnel de nettoyage, conforme à l'une des revendications précédentes, qui ne contient aucune huile de type ester de synthèse.

15. Utilisation d'une composition de soin personnel de nettoyage, conforme à l'une des revendications précédentes, pour le nettoyage de la peau et/ou des cheveux.
